# EUROPÄISCHE PATENTSCHRIFT

(11) **EP 1 964 570 B1**
(45) Veröffentlichungstag und Bekanntmachung des Hinweises auf die Patenterteilung: **21.11.2012**
(21) Anmeldenummer: 07004329.4
(22) Anmeldetag: 02.03.2007
(51) Int. Cl.: A61K 35/74, A61P 37/08, A61P 3/10, A61P 17/00, A61P 29/00

(54) **Pharmazeutische Zusammensetzung zum Schutz vor Allergien und entzündlichen Erkrankungen**
Pharmaceutical compound to protect against allergies and inflammatory illnesses
Composition pharmaceutique destinée à la protection contre les allergies et les maladies infectieuses

(43) Veröffentlichungstag der Anmeldung: 03.09.2008
(73) Patentinhaber: Protectimmun GmbH, 45886 Gelsenkirchen (DE); Forschungszentrum Borstel, 23845 Borstel (DE)
(72) Erfinder: Bauer, Johann, 85737 Ismaning (DE); Blümer, Nicole, 35059 Marburg (DE); Bufe, Albrecht, 20255 Hamburg (DE); Debarry, Jennifer, 30451 Hannover (DE); Ege, Markus, 80333 München (DE); Friedrich, Susanne, 44143 Dortmund (DE); Garn, Holger, 35037 Marburg (DE); Gatermann, Sören, 44799 Bochum (DE); Hanuszkiewicz, Anna, 41100 Siemianowice Slaskie (PL); Heine, Holger, 23867 Sülfeld (DE); Holst, Otto, 23843 Oldesloe (DE); Renz, Harald, 35043 Marburg (DE); Von Mutius, Erika, 82319 Leutstetten/Starnberg (DE); Wegmann, Michael, 35091 Cölbe (DE)
(74) Vertreter: Polypatent

(56) Entgegenhaltungen:
- EP-A- 0 904 784
- EP-A2- 0 178 443
- WO-A-2005/030230
- DE-U1- 20 202 562
- DATABASE WPI Week 199812 Derwent Publications Ltd., London, GB; AN 1998-126086 XP002448490 & JP 10 007577 A (YAKULT HONSHA KK) 13. Januar 1998 (1998-01-13)
- OUWEHAND ARTHUR C: "Antiallergic effects of probiotics." THE JOURNAL OF NUTRITION MAR 2007, Bd. 137, Nr. 3 Suppl 2, März 2007 (2007-03), Seiten 794S-7S, XP009088742 ISSN: 0022-3166
- WU ET AL: "Immunomodulatory effects of IL-12 secreted by Lactococcus lactis on Th1/Th2 balance in ovalbumin (OVA)-induced asthma model mice" INTERNATIONAL IMMUNOPHARMACOLOGY, ELSEVIER, AMSTERDAM, NL, Bd. 6, Nr. 4, April 2006 (2006-04), Seiten 610-615, XP005309031 ISSN: 1567-5769
- DATABASE WPI Week 198208 Derwent Publications Ltd., London, GB; AN 1982-14903E XP002448491 & JP 57 009722 A (TEIJIN LTD) 19. Januar 1982 (1982-01-19)
- DATABASE WPI Week 200019 Derwent Publications Ltd., London, GB; AN 2000-128888 XP002448603 & JP 2000 044480 A (null) 15. Februar 2000 (2000-02-15)

### SEQUENZPROTOKOLL

<110> Bufe et al., Albrecht
<120> Pharmazeutische Zusammensetzung zum Schutz vor Allergien und entzündlichen Erkrankungen
<130> P13064EPSEQ
<140> 07004329.4
   <141> 2007-03-02
<160> 20
<170> PatentIn Ver. 2.1
<210> 1
   <211> 16
   <212> DNA
   <213> Künstliche Sequenz
<220>
   <223> Beschreibung der künstlichen Sequenz:Primer
<400> 1
   acacatgcaa gtcgag 16
<210> 2
   <211> 17
   <212> DNA
   <213> Künstliche Sequenz
<220>
   <223> Beschreibung der künstlichen Sequenz:Primer
<400> 2
   ctacttctgg tgcaaca 17
<210> 3
   <211> 16
   <212> DNA
   <213> Künstliche Sequenz
<220>
   <223> Beschreibung der künstlichen Sequenz:Primer
<400> 3
   cctaatacat gcaagt 16
<210> 4
   <211> 17
   <212> DNA
   <213> Künstliche Sequenz
<220>
   <223> Beschreibung der künstlichen Sequenz:Primer
<400> 4
   gctaccttgt tacgact 17
<210> 5
   <211> 16
   <212> DNA
   <213> Künstliche Sequenz
<220>
   <223> Beschreibung der künstlichen Sequenz:Primer
<400> 5
   accggctaac tctgtg 16
<210> 6
   <211> 17
   <212> DNA
   <213> Künstliche Sequenz
<220>
   <223> Beschreibung der künstlichen Sequenz:Primer
<400> 6
   ttcaccgcta cacctgg 17
<210> 7
   <211> 16
   <212> DNA
   <213> Künstliche Sequenz
<220>
   <223> Beschreibung der künstlichen Sequenz:Primer
<400> 7
   tgcattggaa actggt 16
<210> 8
   <211> 17
   <212> DNA
   <213> Künstliche Sequenz
<220>
   <223> Beschreibung der künstlichen Sequenz:Primer
<400> 8
   gtgcatggtt gtcgtca 17
<210> 9
   <211> 16
   <212> DNA
   <213> Künstliche Sequenz
<220>
   <223> Beschreibung der künstlichen Sequenz:Primer
<400> 9
   ggaatagcac gagtat 16
<210> 10
   <211> 16
   <212> DNA
   <213> Künstliche Sequenz
<220>
   <223> Beschreibung der künstlichen Sequenz:Primer
<400> 10
   tcagttacag gccaga 16
<210> 11
   <211> 16
   <212> DNA
   <213> Künstliche Sequenz
<220>
   <223> Beschreibung der künstlichen Sequenz:Primer
<400> 11
   gtcaggcagt ataatc 16
<210> 12
   <211> 16
   <212> DNA
   <213> Künstliche Sequenz
<220>
   <223> Beschreibung der künstlichen Sequenz:Primer
<400> 12
   gggcatatcc tacaac 16
<210> 13
   <211> 17
   <212> DNA
   <213> Künstliche Sequenz
<220>
   <223> Beschreibung der künstlichen Sequenz:Primer
<400> 13
   cctgtagccc atgttgt 17
<210> 14
   <211> 16
   <212> DNA
   <213> Künstliche Sequenz
<220>
   <223> Beschreibung der künstlichen Sequenz:Primer
<400> 14
   ttgaagagga cctggg 16
<210> 15
   <211> 16
   <212> DNA
   <213> Künstliche Sequenz
<220>
   <223> Beschreibung der künstlichen Sequenz:Primer
<400> 15
   aaaggaagat ggaatt 16
<210> 16
   <211> 17
   <212> DNA
   <213> Künstliche Sequenz
<220>
   <223> Beschreibung der künstlichen Sequenz:Primer
<400> 16
   tgctgctttt gacactg 17
<210> 17
   <211> 17
   <212> DNA
   <213> Künstliche Sequenz
<220>
   <223> Beschreibung der künstlichen Sequenz:Primer
<400> 17
   ggcctgtttt gtgacca 17
<210> 18
   <211> 16
   <212> DNA
   <213> Künstliche Sequenz
<220>
   <223> Beschreibung der künstlichen Sequenz:Primer
<400> 18
   cgacaggtgc aggtgt 16
<210> 19
   <211> 16
   <212> DNA
   <213> Künstliche Sequenz
<220>
   <223> Beschreibung der künstlichen Sequenz:Primer
<400> 19
   ctggcggctg ggaagg 16
<210> 20
   <211> 16
   <212> DNA
   <213> Künstliche Sequenz
<220>
   <223> Beschreibung der künstlichen Sequenz:Primer
<400> 20
   gagggctgca gtcatt 16

## Beschreibung

Die vorliegende Erfindung betrifft eine pharmazeutische Zusammensetzung, die natürlich vorkommende, nicht transgene isolierte Bakterien aus der Gruppe Lactococcus und Acinetobacter oder Fragmente davon oder Mischungen daraus enthält eine solche Mischung als Medikament zur Vorbeugung und/oder Behandlung von allergischen oder chronisch entzündlichen Erkrankungen und ein Verfahren zur Herstellung dieser Zusammensetzung.

Als Allergien bezeichnet man überschießende Reaktionen des Körpers, insbesondere des Immunsystems, auf nicht schädliche fremde Substanzen. Diese Reaktionen laufen genauso wie die normale Immunantwort gegen einen Krankheitserreger ab. Es werden mehrere Typen der allergischen Immunantwort unterschieden. Allergische Reaktionen vom Typ 1, zu denen bronchiales Asthma, Atopische Dermatitis, Urtikaria (Nesselsucht), Heuschnupfen sowie Nahrungsmittelallergien gehören, sind am weitesten verbreitet. Obgleich diese Krankheiten unterschiedliche körperliche Symptome aufweisen, liegen Ihnen ähnliche immunologische Mechanismen zugrunde.

Zahlreiche Studien belegen, dass allergische Erkrankungen zunehmen. Die Ursachen der Entstehung und Entwicklung der Allergien sind bisher unklar. Bestimmte bisher unbekannte Erbfaktoren und Umweltbedingungen wie Allergenexposition, Leben in der Stadt, Anzahl der Geschwister und manche Infektionen tragen wahrscheinlich zur Entstehung bei.

Allergien vom Typ 1 werden durch Antikörper der Gruppe E vermittelt, deren Entstehung von T-Heiferlymphozyten der Gruppe II abhängig ist. Diese Th2-Zellen kommen bei Allergien im Verhältnis zu den Th1 Zellen häufiger vor. Kinder werden zunächst mit einer Th2 dominierten Immunantwort geboren, die sich im ersten Lebensjahr wohl unter dem Einfluß der mikrobiellen Belastung zu einer Th1 dominierten Antwort (nicht allergischen Immunantwort) verschiebt. Es wird angenommen, dass häufige Infektionen mit einer geringeren Th2 Antwort und damit weniger Allergien korrelieren. Eine niedrige mikrobielle Belastung könnte also zum verstärkten Auftreten von Allergien führen (Hygienehypothese).

Die Möglichkeiten zur Prävention und Therapie allergischer Erkrankungen sind begrenzt. Die Symptome lassen sich zwar relativ gut durch zahlreiche Medikamente lindern, die immuntherapeutischen Behandlungen wie die Desensibilisierung sind aber unterschiedlich erfolgreich. Beispielsweise bei dem häufig auftretenden bronchialen Asthma ist diese Therapie eher unwirksam. Auch die Allergenvermeidung als präventive Maßnahme trägt nicht sicher zur Senkung der Allergiehäufigkeit bei. Insgesamt gibt es keine Möglichkeiten, dass Risiko für Allergieentstehung zu senken.

Die WO 01/49319 beschreibt eine Zusammensetzung, die auf/in Mikroorganismen befindliche Antigene enthält und deren Benutzung zur Vorbeugung und Behandlung von allergischen Erkrankungen. Diese Zusammensetzung wird dadurch hergestellt, dass Stallstaub gesammelt wird und dieser gegebenenfalls in einem geeigneten Lösungsmittel, z.B. Wasser oder isotonischer Salzlösung suspendiert wird. Somit wird eine Suspension von Mikroorganismen oder auch Fragmenten dieser Organismen in einem Lösungsmittel hergestellt, die direkt oder nach weiteren Behandlungsschritten zur Behandlung von Allergien verabreicht werden kann.

Die EP-A 1 637 147 beschreibt einen Extrakt aus Stallstaub zur Behandlung von allergischen Erkrankungen, in dem während des gesamten Herstellungsverfahrens keine Wärmezufuhr erfolgt, ein Verfahren zu dessen Herstellung und die Verwendung dieses Extraktes zur Herstellung eines Medikamentes zur Behandlung von allergischen Erkrankungen.

Die WO 96/00579 beschreibt ein Herstellungsverfahren zum Herstellen einer Suspension und eines Extraktes von Mycobakterien in wässriger Lösung, bei dem der Extrakt wenigstens 20 Minuten auf 121°C erhitzt wird. Diese Suspension oder dieser Extrakt kann für die unspezifische Immunmodulation eingesetzt werden.

Die Verwendung von Lactococcus species wird in erster Linie zur oralen Aufnahme für probiotische Zwecke beschrieben (beispielsweise Kimoto et al., Microbiol Immunol. 2004; 48(2):75-82; Perdigon, G. et al., Int J Immunopathol Pharmacol. 1999 May; 12(2):97-102; Villamil, L. et al., Clin Diagn Lab Immunol., 2002 Nov; 9(6):1318-23; Vitini E. et al., Biocell. 2000 Dec; 24(3):223-32; Perdigon, G. et al. J Diary Sci., 1999 Jun; 82(6):1108-14; Huis in't Veld JH., Ned Tijdschr Tandheelkd. 1992 Dec; 99(12):467-70), aber auch als Träger für das Einbringen rekombinanter Gene, deren Expression Krankheitszustände mildem können (Cortes-Perez, N. et al., Clin. Vaccine Immunol. Published online on 3.January 2007; Daniel, C. et al., Allergy, 2006 Jul; 61(7):812-9; DE-A 101 01 793).

Der Einsatz von Lactocoocus sp. zur Prävention und zur Behandlung von Erkrankungen ist ebenfalls beschrieben, und zwar bei oraler Aufnahme zur Prävention und Behandlung von Übergewicht oder Diabetes (KR1020010106068), von Magenerkrankungen (KR1020040044300) und von rheumatischer Arthritis (EP-A . 762 881). Ein Antigen-Antitoxin-Gemisch zur Herstellung eines homöopatischen Präparates, dessen Hauptanwendung auf den Gebieten der Herz-/Kreislauf störungen, Hypertonie und allergischen Leiden liegt, wird in der DE-A 100 07 771 beschrieben.

Die Aufgabe der vorliegenden Erfindung war es ein schonendes Mittel zur Vorbeugung und Behandlung von allergischen Erkrankungen und/ oder entzündlichen Erkrankungen bereitzustellen.

Diese Aufgabe wird gelöst durch eine pharmazeutische Zusammensetzung zur nasalen oder inhalativen Verabreichung. enthaltend natürlich vorkommende, nicht transgene, isolierte Bakterien, ausgewählt aus der Gruppe bestehend aus Lactococcus und Acinetobacter oder Fragmente davon oder eine Mischung davon und ggf. einen pharmazeutisch akzeptablen Träger zur Vorbeugung und / oder Behandlung von allergischen oder chronischen entzündlichen Erkrankungen, ausgewählt aus IgE-abhängigen Typl Allergien oder Typ IV Allergien

Ein Gesichtspunkt der vorliegenden Erfindung ist der Einsatz von Bakterien der Species Lactococcus und/oder Acinetobacter als natürlich vorkommende, nicht gentechnisch veränderte, insbesondere nicht transgene Organismen. Ein weiterer Gesichtspunkt ist der Einsatz der Bakterien in isolierter Form. Dies bedeutet, dass entweder eine Lactococcus Spezies, bevorzugt Lactococcus lactis, besonders bevorzugt Lactococcus lactis, Stamm G121, und/oder eine Acinetobacter Spezies, bevorzugt Acinetobacter Iwoffii, besonders bevorzugt Acinetobacter Iwoffii, Stamm F78 oder eine Mischung dieser beiden Bakterien Spezies in zuvor isolierter und ggf. gereinigter Form in die pharmazeutische Zusammensetzung eingesetzt werden.

Die genannten Bakterien Können als Mischungen auch unmittelbar als Medikament zur Vorbeugung und / oder Behandlung von allergischen oder chronischen entzündlichen Erkrankungen, ausgewählt aus IgE-abhängigen Typl Allergien oder Typ IV Allergien und chronisch entzündlichen Hauterkrankungen oder Autoimmunerkrankungen eingesetzt werden.

In einer weiteren Ausführungsform der Erfindung werden Fragmente dieser Bakterien eingesetzt. Unter Fragmenten im Sinne dieser Erfindung sind bevorzugt Membranstücke und Membranbestandteile, Zellwandproteine, insbesondere glycosylierte Proteine, Polysaccharide, Lipopolysaccharide (Endotoxine), cytosolische Proteine, von den Bakterien sezemierte Moleküle und/oder Verbindungen und/oder Metabolite, Nukleinsäuren und/oder Organelle zu verstehen. Die Fragmente der Bakterien können auch gleichzeitig mit vollständigen Bakterien derselben oder der anderen Spezies eingesetzt werden, also im Gemisch mit diesen, oder es können von einer Spezies vollständige Bakterien und von der anderen Spezies Fragmente eingesetzt werden.

Die (unfragmentierten) Bakterien können in vitalem Zustand oder als abgetötete Bakterien als Medikament verwendet oder in die pharmazeutische Zusammensetzung eingesetzt werden. Da die genannten Bakterien selbst für Säugetierorganismen vollkommen harmlos sind, ist ein Einsatz in vitalem Zustand unproblematisch und entspricht einer bevorzugten Ausführungsform der Erfindung. Um eine Verkeimung der pharmazeutischen Zusammensetzung mit anderen, nicht so harmlosen Mikroorganismen zu vermeiden, ist aber auch eine Sterilisierungsmethode ohne weiteres anwendbar, wie z.B. das Autoklavieren, das einfache Abkochen oder Erhitzen, der Einsatz von Bakteriziden, Bakteriostatika, Fungiziden, Fungistatika, Viriziden und/oder Virostatika, UV-Bestrahlung oder die Verwendung von Lösungsmitteln, die für die Bakterien unverträglich sind, wie z.B. Alkoholen, insbesondere Ethanol, Propanol, Isopropanol usw., Lyophilisieren oder Kältesterilisation.

Ein besonderer Vorteil der vorliegenden Erfindung ist die hohe Wirksamkeit bei sehr einfachem Wege der Verabreichbarkeit der erfindungsgemäßen Mischung . Die Verabreichung kann oral, nasal, konjunktival, inhalativ, subkutan, intraartikulär, intraperitoneal, rektal oder vaginal erfolgen. Bevorzugt ist die nasale oder inhalative Verabreichung.

Eine pharmazeutische Zusammensetzung gemäß der Erfindung kann als Aerosol, Lösung, bevorzugt wässrige Lösung oder wässrig-alkoholische Lösung, Suspension, Lyophilisat oder Pulver vorliegen. Alle diese Ausführungsformen eigenen sich besonders gut für die nasale, orale oder inhalative Verabreichung der Bakterien und /oder Fragmenten daraus.

Ein erfindungsgemäßes Aerosol besteht bevorzugt aus kleinen festen oder flüssigen Teilchen, die durch einen Inhalator, einen Vernebler oder ein Beatmungsgerät erzeugt werden können. Die Teilchen des Aerosols können aus der Zusammensetzung allein oder der Zusammensetzung in Verbindung mit einem geeigneten Trägermaterial bestehen. Das Aerosol kann beispielsweise eine Teilchengröße von bis zu 100µm für die installative nasale Anwendung haben. Für eine inhalative Anwendung kann das Aerosol beispielsweise Teilchen einer Teilchengröße von bis zu 10µm, bevorzugt von unter 5µm enthalten.

Eine erfindungsgemäße Lösung, enthaltend beispielsweise lösliche Fragmente der genannten Bakterien, kann bevorzugt eine wässrige Lösung oder eine wässrig-alkoholische Lösung, beispielsweise ein Gemisch von Wasser oder einer wässrigen Lösung mit Ethanol, Propanol oder Isopropanol sein. Die Lösung kann beispielsweise gepuffert oder salzhaltig, bevorzugt isotonisch sein, d.h. Puffermoleküle oder eine physiologische Salzkonzentration enthalten. Die Lösung kann außerdem unbedenkliche, z.B. natürliche oder synthetische Zusatzstoffe oder Hilfsstoffe enthalten, wie Konservierungsstoffe, Stabilisatoren, Geruchs- und Geschmacksbildner, z. B. Zucker, pharmazeutisch unbedenkliche Trägerstoffe, Emulgatoren, Verdünnungsmittel und wenn gewünscht unbedenkliche Farbstoffe.

Im Falle der Anwendung vollständiger Bakterien oder unlöslicher Bruchstücke und Bestandteile daraus, können diese in Form einer Suspension vorliegen. Für das Suspendieren kann eine Lösung verwendet werden, wie sie zuvor beschrieben ist oder jedes andere geeignete Suspendiermedium.

Die Bakterien können auch in Form eines Lyophilisats vorliegen, oder zu einem Pulver verarbeitet oder in ein pharmazeutisch verabreichbares Pulver eingearbeitet werden. Ein solches Lyophilisat oder Pulver kann mit weiteren pharmazeutisch akzeptablen Bestandteilen versetzt werden, insbesondere da es über einen Pulverinhalator verabreicht werden kann.

In einer weiteren bevorzugten Ausführungsform der Erfindung wird eine Mischung von Lactococcus und Acinetobacter Spezies, bevorzugt eine Mischung der oben genannten Spezies und / oder Stämme als Medikament verwendet oder in eine pharmazeutische Zusammensetzung eingesetzt. Im Falle dieser Ausführungsform beträgt das Mischungsverhältnis der eingesetzten Bakterienspezies Lactococcus : Acinetobacter von 5:95 bis 95:5, bevorzugt 40:60 bis 60:40, besonders bevorzugt 50:50. Die Verhältnisse sind als ca..- Angaben zu verstehen.

In einer weiteren Ausführungsform der Erfindung können Lactococcus und/oder Acinetobacter Bakterien auch in Kombination mit einer oder mehreren Spezies der folgenden Bakterienarten eingesetzt werden: Micrococcus spp., Planococcus spp., Staphylococcus spp., Streptococcus spp., Lactococcus spp., Aerococcus spp., Gemella spp., Enterococcus spp., Bacillus spp., Clostridium spp., Micromonospora spp., Lactobacillus spp., Listeria spp., Erysipelothrix spp., Acinetobacter spp., Leuconostoc spp., Corynebacterium spp., Clavibacter spp., Brevibacterium spp., Propionibacterium spp., Arthrobacter spp., Curtobacterium spp., Microbacterium spp., Aureobacterium spp., Cellulomonas spp., Agromyces spp., Eubacterium spp., Pseudomonas spp., Xantomonas spp., Frateuria spp., Zoogloea spp., Escherichia spp., Enterobacter spp., Pantoea spp., Erwinia spp., Citrobacter spp., Klebsiella spp., Proteus spp., Serratia spp., Actinomyces spp., Streptomyces spp., Nocardia spp., Mycobacterium spp., Absidia spp., Wallemia spp., Eurotium spp., Acremonium spp., Aspergillus spp., Penicillium spp., Cladosporium spp., Cephalosporium spp., Fusarium spp., Mucor spp., Trichoderma spp., oder in Kombination mit Trichothecenen, Fumonisinen, Gliotoxin, Ochratoxinen, Patulin, Aflatoxinen.

Ein besonderer Vorteil der Verwendung der hier genannten Bakterien ist aufgrund deren eigener Harmlosigkeit, dass eine besonders schonende Prävention oder Behandlung durchgeführt werden kann. Die genannten Bakterien kommen in der Umwelt ubiquitär vor und es ist kein negativer Einfluss auf das körperliche Wohlbefinden oder auf die Gesundheit bekannt. Bei Einsatz der Bakterien vor dem Auftreten einer allergischen oder entzündlichen Erkrankung kann einer solchen Krankheit vorgebeugt werden, oder die Heftigkeit des Ausbruchs gemildert werden. Dies wird allgemein als "Prävention" bezeichnet. Bei erfindungsgemäßer Verwendung der genannten Bakterien kann eine solche Prävention oder auch eine Behandlung bereits im Säuglingsalter, oder eine Prävention durch Gabe an Schwangere bereits im Mutterleib durchgeführt werden. Insbesondere bei Kenntnis einer familiären Veranlagung für Allergien oder (chronisch) entzündliche Erkrankungen ist die vorliegende schonende Präventions- und Behandlungsmöglichkeit besonders zu bevorzugen.

Die vorliegende Erfindung ist geeignet insbesondere das noch nicht geprägte (naive) und das kindliche Immunsystem zu beeinflussen. Daher kann die Zusammensetzung oder das Medikament erfindungsgemäß Schwangeren, Säuglingen und Kindern in den ersten Lebensjahren wiederholt verabreicht werden. Bevorzugt wird die Zusammensetzung oder das Medikament Schwangeren, Säuglingen oder Kindern verabreicht, die aufgrund einer positiven Familienanamnese ein deutlich erhöhtes Risiko aufweisen eine allergische Erkrankung zu entwickeln. Eine positive Familienanamnese kann angenommen werden, wenn wenigstens ein Elternteil und/oder wenigstens ein Geschwister oder ein anderer naher Verwandter bereits an einer allergischen oder chronisch entzündlichen Erkrankung leidet oder deren Symptome zeigt.

Die erfindungsgemäße Zusammensetzung oder das Medikament kann auch zur Behandlung für Schwangere, Säuglinge und Kinder eingesetzt werden, die bereits erste Anzeichen einer allergischen oder chronisch entzündlichen Erkrankung aufweisen. Ein erhöhtes Risiko für eine Erkrankung kann auch genetisch nachgewiesen werden.

Das Medikament oder die Zusammensetzung kann zur Behandlung oder Prävention von Kindern und Erwachsenen verabreicht werden. Besonders bevorzugt kann das Medikament oder die Zusammensetzung zur Prävention an Schwangere, Säuglinge oder Kinder, insbesondere in den ersten Lebensjahren, bevorzugt in den ersten 5 Lebensjahren, besonders bevorzugt in den ersten 2 Lebensjahren verabreicht werden.

Die Zusammensetzung wird bevorzugt mit einem üblichen Inhalator, Zerstäuber, Vernebler oder Beatmungsgerät verabreicht. Sie kann in regelmäßigen Abständen über einen längeren Zeitraum verabreicht werden, um eine kontinuierliche Stimulation im Organismus zu bewirken. Die Zusammensetzung kann beispielsweise bis zu 10 Jahre 1 bis 21 mal wöchentlich, vorzugsweise 7 bis 14mal wöchentlich verabreicht werden. Hierbei kann je Behandlung ein einfaches Applizieren (z.B. durch Einspritzen mittels eines Zerstäubers in die Nase) erfolgen, oder z.B. ein längeres Einatmen. Im letztgenannten Fall kann die Dauer jeder Behandlung zwischen 1 bis 120 min, bevorzugt zwischen 5 bis 60 min liegen.

Erkrankungen, denen auf diese schonende und harmlose Weise vorgebeugt werden kann, oder die auf diese Weise behandelt werden können, sind allergische oder chronische entzündlichen Erkrankungen, ausgewählt aus 1gE-abhängigen Typl Allergien oder Typ IV Allergien und chronisch entzündlichen Hauterkrankungen oder Autoimmunerkrankungen, beispielsweise Heuschnupfen, Nahrungsmittelallergien, Asthma, Neurodermitis, atopische Dermatitis, Kontaktekzem, Psoriasis, Diabetes Typ 1 und 2, multiple Sklerose, Kollagenosen, Rheumatoide Arthritis, Schilddrüsenerkrankungen wie Hashimoto Thyreoditis und Graves disease.

Für die erfindungsgemäße pharmazeutische Zusammensetzung oder die Verwendung der genannten Bakterien als Medikament spielt die Herkunft der Bakterien keine Rolle. Die Bakterien können aus einer natürlichen Quelle wie Gras, Heu, Hausstaub, Stallstaub, Nahrungsmitteln oder ähnlichem isoliert, ggf. gereinigt und in Kultur angezüchtet werden, oder sie können kommerziell als Einzelkulturen erworben werden.

### Hersteilungsverfahren

Zur Herstellung eines pharmazeutischen Zusammensetzung gemäß der Erfindung werden Lactococcus Bakterien und/oder Acinetobacter Bakterien, bevorzugt Lactococcus lactis und/oder Acinetobacter Iwoffii, besonders bevorzugt Lactococcus lactis, G121 und/oder Acinetobacter Iwoffii F78 entweder aus einer natürlichen Quelle isoliert oder kommerziell erworben und in Kultur gezüchtet. Beispielsweise können die Bakterien aus Stallstaub isoliert werden und unter geeigneten Kultivierungsbedingungen herangezogen werden, wie es in Beispiel 1 dargestellt ist.

Nach der Ernte der Bakterien können diese entweder unmittelbar in einem wässrigen oder wässrig-alkoholischen Medium aufgenommen werden, um eine Bakteriensuspension zu erhalten, oder sie können zuvor einen Fragmentierungsschritt durchlaufen. Geeignete Verfahren zur Fragmentierung sind den Fachleuten auf diesem Gebiet bekannt und umfassen beispielsweise chemische Lyseverfahren, Druck/Entspannung (z.B. unter Verwendung der sogenannten French Press), mechanische Verfahren oder Ultraschall, um einige Aufschlussverfahren zu nennen. Es kann sich hieran auch ein spezifisches Aufreinigungsverfahren anschließen, beispielsweise wenn einige Zellbestandteile in der pharmazeutischen Zusammensetzung unerwünscht sind. Beispielsweise können Nukleinsäuren selektiv abgetrennt werden.

Alternativ zur Fragmentierung, oder auch ergänzend hierzu kann ein Schritt durchgeführt werden, der ein Abtöten der Bakterien bewirkt. Hierzu kann jedes bekannte und geeignete Verfahren angewendet werden, wie z.B. das Autoklavieren, Abkochen, Anlegen von Hitze, Strahlung (UV, radioaktive), der Einsatz von Bakteriziden, Bakteriostatika, Fungiziden, Fungistatika, Viriziden und/oder Virostatika, oder die Verwendung von Lösungsmitteln, die für die Bakterien unverträglich sind, wie z.B. Alkoholen, insbesondere Ethanol, Propanol, Isopropanol usw oder eine Kältesterilisation

Die aus den zuvor genannten Schritten erhaltenen vitalen oder abgetöteten Bakterien oder die daraus erhaltenen Fragmente können unmittelbar als Medikament verwendet werden, in ein solches eingearbeitet werden, oder mit (einem) pharmazeutischen Träger(n) und Hilfsmittel(n) versetzt werden, um sie in Form eines Aerosols, einer Lösung oder einer Suspension bereitzustellen.

Ein besonders bevorzugter Verfahrensschritt, der optional durchgeführt werden kann, ist das Trockenen oder das Lyophilisieren (Gefriertrocknen) der vitalen oder abgetöteten Bakterien oder der daraus erhaltenen Fragmente. Beim Lyophilisieren können zur Stabilisierung auch beispielsweise Saccharose, Dextran oder Glycerin zugefügt werden. Das so entstehende Trockenprodukt oder Lyophilisitat kann dann unmittelbar als Medikament eingesetzt werden oder mit pharmazeutisch verträglichen Hilfsmitteln versetzt werden und beispielsweise zu einem inhalierbaren Pulver verarbeitet werden.

Der pharmazeutisch akzeptable Träger kann Wasser, eine Puffer- oder Salzlösung, bevorzugt eine physiologische Kochsalzlösung, ein Alkohol oder eine wässrig-alkoholische Mischung sein, oder ein pharmazeutisch akzeptables Pulver, wie z.B. ein fein gemahlenes Silikat, Stärke, Cellulose und Cellulosederivate und vieles mehr. Die Anwendung erfolgt unter anderem als Aerosol mittels eines Vemeblers oder Zersteubers, einem Druckgasaerosol mittels eines "vibrating orifice generators" oder eines "spinning disk generators" oder einer Pulverinhalation mittels gängiger Zerstäubersysteme wie Ein- oder Zweistoffdüsen.

### Abbildungen:

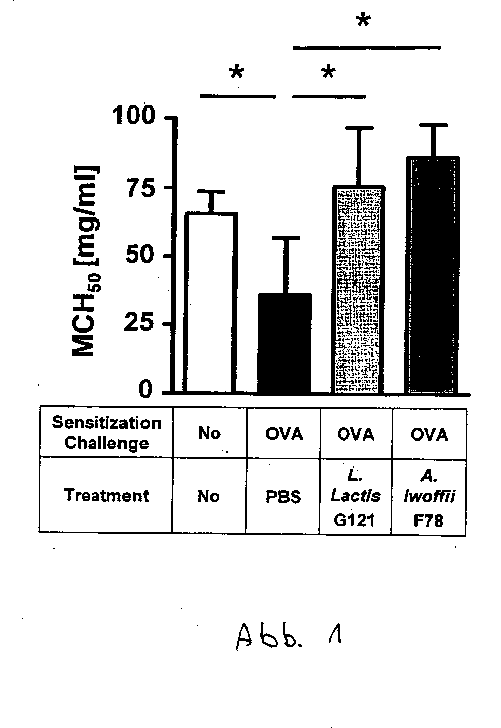

**Abb. 1****:** Intranasale Behandlung mit L.lactis G121 und A.Iwoffü F78 verbessert die Reaktivität der Luftwege gegenüber Methacholin bei allergischen Mäusen. Die Mäuse wurden wie unten unter 2.2. beschrieben behandelt. Die Lungenfunktion wurde wie beschrieben analysiert. Es ist eine mittlere signifikante Differenz (±SD) der Methacholin-Konzentration angegeben, die eine 50%ige Reduktion des mittleren Ausatmungs-Luftflusses (MCH₅₀) bei acht Mäusen pro Gruppe bewirkte. Signifikante Unterschiede mit p<0,05 sind durch ein * gekennzeichnet

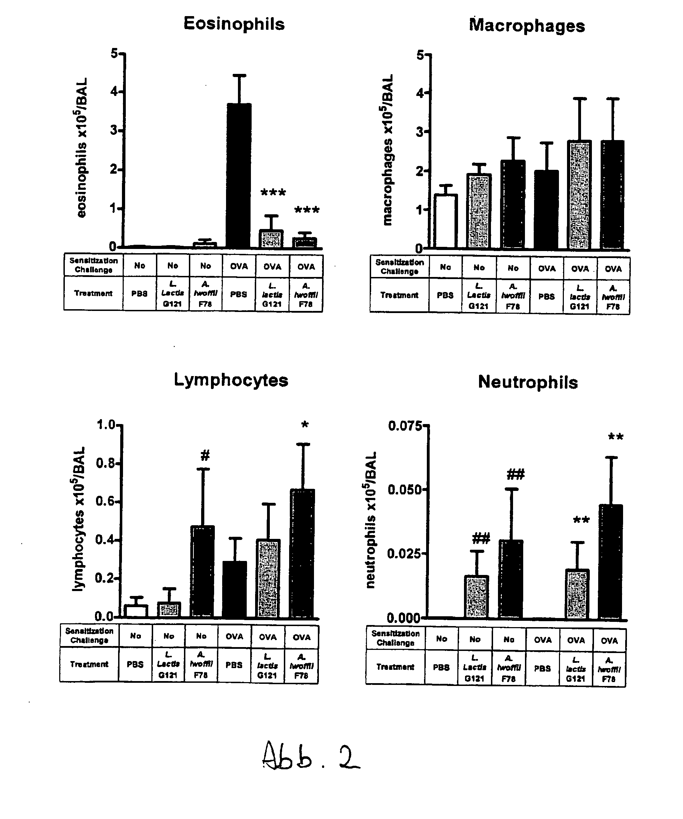

Abb. 2: Inhibierung der Eosinophile-Infiltration durch i.n. Behandlung von Mäusen mit L.lactis G121 und A.Iwoffi F78. Gruppen von Mäusen (je n=6) wurden i.n. entweder mit PBS, L. lactis G121 oder A.lwoffii F78 behandelt, sensibilisiert und gegenüber OVA ausgesetzt, oder verblieben unsensibilisiertlohne OVA, wie es unten unter 2.3. beschrieben ist. Die Anzahl der Eosinophilen, Lymphozyten, Makrophagen und Neutrophilen wurde in der BAL durch mikroskopische Bestimmung analysiert Die Ergebnisse sind als mittlere signifikante Differenz (±SD) dargestellt. Signifikante Differenzen gegenüber der nicht sensibilisierten PBS-behandelten Kontrollgruppe sind durch # angezeigt, gegenüber der sensibilisierten PBS-behandelten Gruppe durch ein * mit p<0,05 (#,*), p<0,01 (##,**) oder p<0,001 (***).

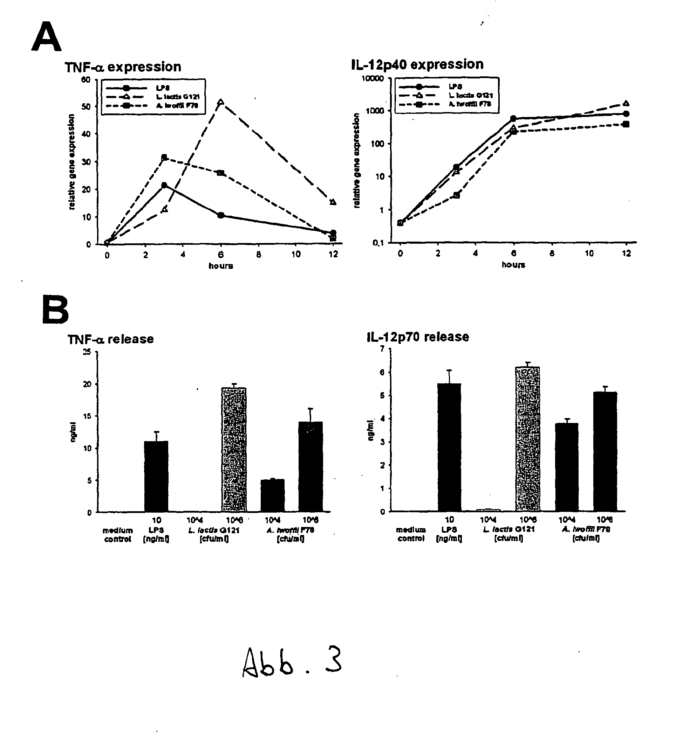

**Abb. 3****:** Bakterien-induziertes Cytokin-Muster in menschlichen moDCs. (A) Menschllche moDCs wurden für die jeweils angegebene Zeitdauer mit LPS (10ng/ml (Kreise)), mit lebenden-L.lactis G121 (10⁶cfu/ml, Dreiecke)) und lebenden A.Iwoffii F78 (10⁶ cfu/ml, Quadrate)) stimuliert. Es wurde die TNF-α und IL-12p40 mRNA im Verhältnis zu HPRT durch quantitative real-time PCR bestimmt. Die Daten stellen ein Mittel von drei unabhängigen Experimenten mit moDCs aus unterschiedlichen gesunden Spendern dar. (B) Behandlung menschlicher moDCs mit LPS und ansteigenden Mengen hitzeinaktivierter Bakterien. Die TNF-α und IL-12p70 Freisetzung wurde nach 24 Std Inkubation im Kulturüberstand gemessen.

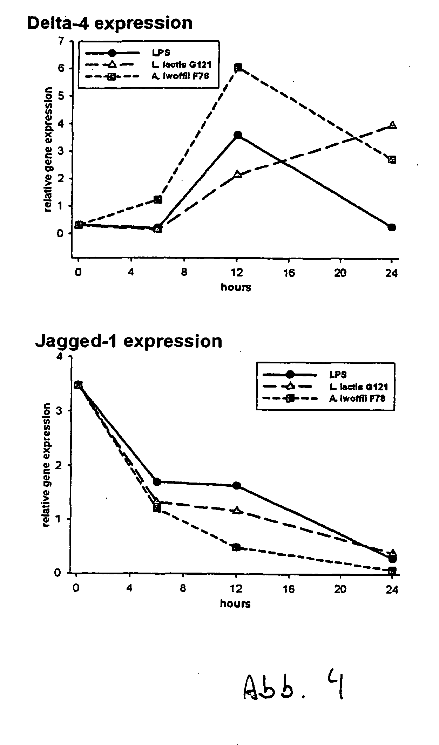

**Abb. 4****:** Beide Bakterienarten induzieren eine T_{H}1-polarisierende Notch-Liganden-Expression in menschlichen moDCs. Meschliche moDCs wurden für die angegebene Zeitdauer mit LPS (10ng/ml (Kreise)), mit lebenden L.lactis G121 (10⁸cfu/ml, Dreiecke)) und lebenden A.lwoffii F78 (10⁶ cfu/ml, Quadrate)) stimuliert. Delta-4 und Jagged-1 mRNA wurde unter Anwendung der quantitativen real-time PCR gemessen und die Daten sind als mRNA-Mengen beider Gene im Verhältnis zu HPRT dargestellt. Die Daten stellen ein Mittel von drei unabhängigen Experimenten mit moDCs aus unterschiedlichen gesunden Spendern dar.

### Beispiele:

### Beispiel 1 : Isolierung und Charakterisierung der Stämme:

Die Bakterienstämme L.lactis G121 und A.lwoffii F78 wurden nach dem Kultivieren von Staubproben, die aus Viehställen aus Bayern, Deutschland gesammelt worden waren, isoliert und weiter kultiviert. Acinetobacter Iwoffi F78 wurde in SB Medium (10 g Mops, 20 g Hefeextrakt, 30 g Trypton in 1 Liter, nach dem Autoklavieren mit 10 ml 2 M Glucose vervollständigt) und Lactococcus lactis G121 in TSB Medium, das 0,3% Hefeextrakt enthielt, herangezogen. Die Kulturbedingungen für beide Stämme waren 30°C bis zur gewünschten Zelldichte (innerhalb der logarithmischen Phase).

Die Charakterisierung der Stämme wurde mit Hilfe einer spezifischen PCR zur Sequenzierung der 16S rDNA durchgeführt. Die PCR Primer wurden auf Basis von konservierten Regionen der A.lwoffii F78 und L.lactis G121 ausgewählt. Für die Amplifikation der 16S rDNA wurden einzelne Kolonien in ein PCR Gefäß überführt mit PCR Reaktionsmischung versetzt (35 µl H₂O, 5µl 10x Advantage 2 PCR Puffer (Clontech, Saint-Germain-en-Laye,Frankreich), 2,5µl jeder Primerlösung (Endkonzentration 10µM/ml), 1 µl jedes dNTPs (dNTP set PCR grade; Invitrogen, Karisruhe, Deutschland) und 1µl 50x Advantage 2 Polymerase Mix (Clontech)). Die PCR wurde mit folgenden Bedingungen durchgeführt: 1 min 95°C, gefolgt von 40 (A.lwoffii) bzw. 30 (L.lactis) Zyklen mit 20 s bei 95°C, 30 s bei 60°C und 1,5 min bei 72°C. Die Reaktionen wurden mit einem letzten Auffüllschritt bei 72°C für 7 min beendet. Die Ampiifikatinsprodukte wurden auf einem 2% Agarosegel analysiert, aus dem Gel isoliert und gereinigt (Qiaquick PCR purification Kit, Qiagen, Hilden, Deutschland) und sequenziert. Die verwendeten Primer sind Tabelle 1 zu entnehmen. Primer 1, 2, 5 und 6 sind PCR-Primer, Primer 3, 4 und 7 bis 10 Sequenzierprimer.

**Tabelle 1**

| Nummer | Primer | Sequenz (5'→3') |
|---|---|---|
| 1 | 5 Aci 16S | ACACATGCAAGTCGAG |
| 2 | 3 Aci 16S | CTACTTCTGGTGCAACA |
| 3 | Aci 16S | CCTAATACATGCAAGT |
| 4 | Aci 16S | GCTACCTTGTTACGACT |
| 5 | 5 Lacto 16S | ACCGGCTAACTCTGTG |
| 6 | 3 Lacto 16S | TTCACCGCTACACCTGG |
| 7 | Lacto SeqPri | TGCATTGGAAACTGGT |
| 8 | Lacto SeqPri | GTGCATGGTTGTCGTCA |
| 9 | Lacto SeqPri | GGAATAGCACGAGTAT |
| 10 | Lacto SeqPri | TCAGTTACAGGCCAGA |

### Beispiel 2: Effektivität In vivo

Die vollständige protektive Potenz der erfindungsgemäßen Zusammensetzung wird durch immunologische und zellbiologische Funktionsuntersuchungen im standardisierten Tiermodell der Maus *in vivo* bestimmt

Im folgenden werden zunächst die Methoden zur Sensibilisierung und Induktion von allergischem Asthma bei Mäusen mit Ovalbumin, der Behandlung mit Lactococcus uns /oder Acinetobacter Bakterien und der Analyse der allergischen und entzündlichen Reaktionen beschrieben.

***2.1. Sensibilisierung und Induktion von allergischem Asthma bei Mäusen mit Ovalbumin (OVA)-*** Zur Sensibilisierung von Mäusen gegen das Modell-Allergen Ovalbumin wird Aluminiumhydroxid (Pierce) als Adjuvans verwendet. Von diesem Adjuvans ist bekannt, dass es Immunantworten auslöst, die durch die Produktion von Cytokinen wie IL-4, IL-5 und IL-13 charakterisiert sind und durch die Produktion von Antikörpern des Isotyps IgE und IgG1 (entsprechender Isotyp beim Menschen ist IgG4).

Die Sensibilisierung von sechs bis acht Wochen alten Mäusen des Inzuchtstammes Balb/c erfolgt durch intraperitoneale Injektion eines Gemisches von 10µg Ovalbumin (Grade V der Firma Sigma) und 1,5 mg Aluminiumhydroxid in einem Gesamtvolumen von 200µl Puffer (PBS). Insgesamt erfolgt die Injektion dreimal, nämlich am Tag 0, am Tag 14 und am Tag 21.

Um anschließend eine allergische Reaktion in der Lunge der Mäuse hervorzurufen, also das akute allergische Asthma auszulösen, werden die Mäuse am Tag 22, 27 und Tag 28 jeweils 20 Minuten lang mit Ovalbumin-Aerosol behandelt. Hierzu werden die Mäuse in eine Plexiglas-Kammer mit 11 Litern Volumen gesetzt. An die Kammer wird ein Inhalator der Firma Pari angeschlossen, der zuvor mit 1%iger Ovalbuminlösung befüllt wurde, und mit einem Pari-Boy-Turbo Aerosol Generator betrieben wird. Nach der zweiten Applikation von OVA-Aerosol kann bei den Mäusen einige Tage lang Symptome des akuten allergischen Asthmas diagnostiziert werden. Zu den Symptomen gehören:
1) die verstärkte Reaktion gegen Methacholin, die bronchiale Hyperreaktivität
2) die verstärkte Infiltration von eosinophilen Granulozyten und Lymphozyten in das Atemwegslumen
3) die Produktion von Ovalbumin spezifischen Antikörpern der Isotypen IgE und IgG1
4) die Produktion von Interleukin-5 nach in vitro Restimulierung von Lymphozyten mit Ovalbumin

Die Methoden zur Messung der einzelnen Parameter werden im folgenden näher erläutert.

***2.2. Messung der bronchlalen Hyperreaktivität** -* Ein entscheidender diagnostischer Parameter, der beim Menschen gemessen wird, um eine allergisch asthmatische Erkrankung sicher zu diagnostizieren, ist die bronchiale Hyperreaktivität. Hierbei handelt es sich um eine Überempfindlichkeit der glatten Muskulatur der Bronchien gegen unspezifische Reizungen. Zur Diagnose beim Menschen werden geringe Konzentrationen an Histamin appliziert und anschließend der Atemwegswiderstand in einem Plethysmographen gemessen. Asthmatische Personen zeigen eine von der Histaminkonzentration abhängige stärkere Bronchokonstriktion und damit einen höheren Atemwegswiderstand als gesunde Personen.

Ganz ähnlich erfolgt die Messung bei Mäusen. Zur Messung werden die Mäuse in einen Plethysmographen der Firma Buxco in die dafür vorgesehenen Kammern gesetzt. Durch die Kammern fließt ein kontinuierlicher Luftstrom und ein hochempfindlicher Druckabnehmer registriert kurzfristige Druckänderungen in der Kammer. Die Einatmung der Mäuse erzeugt einen Unterdruck und die Ausatmung einen Überdruck in der Kammer. Diese Druckänderung werden in Abhängigkeit von der Zeit kontinuierlich aufgezeichnet und von der Software aus den gemessenen Werten für jeden Atemzyklus der sogenannte Penh-Wert berechnet. Dieser dimensionslose Wert korreliert mit dem Atemwegswiderstand. Bei hyperreaktiven Mäusen erhöht sich der Atemwegswiderstand abhängig von der Methacholin-Konzentration (bei Mäusen nimmt man anstelle von Histamin, Methacholin zur Provokation, da die Bronchien von Mäusen nicht auf Histamin reagieren), was sich durch die Erhöhung des Penh-Wertes ausdrückt.

Die Messung der bronchialen Hyperreaktivität erfolgt 24 Stunden nach der letzten QVA-Aerosol Exposition. Für die Messung der bronchialen Hyperreaktivität werden die Mäuse nach einer Gewöhnungszeit von 10 Minuten im Plethysmographen 7 Minuten lang mit PBS-Aerosol in Kontakt gebracht und dabei der Penh.Wert aufgezeichnet. Anschließend erfolgt die Exposition jeweils 7 Minuten lang mit aufsteigenden Konzentrationen an Methacholin: 6, 12, 25, 50mg/ml. Während jeder Exposition wird der Penh-Wert kontinuierlich gemessen und über Perioden von 30 Sekunden gemittelt. Die Periode in der für eine bestimmte Methacholin-Konzentration der maximale Penh-Wert erreicht wird, wird dann gegen die Methacholin-Konzentration aufgetragen. Hieraus ergibt sich eine Dosis-Wirkungs-Kurve, deren Fläche unter der Kurve umso größer ist, umso stärker die Mäuse auf Methacholin reagieren. Die Fläche unter der Kurve korreliert also mit der Hyperreagibilität der Tiere und wird zur statistischen Auswertung verwendet.

***2.3. Untersuchung der zellulären Zusammensetzung der Broncho-Alveolären-Lavage (BAL)** -* Bei gesunden Probanden bzw. unbehandelten Mäusen können nur sehr wenige bis gar keine eosinophile Granulozyten im Lungengewebe und im Lumen der Atemwege beobachtet werden. Die Leukozyten, die in gesunden Atemwegen vorgefunden werden, sind hauptsächlich Makrophagen. Im Zusammenhang mit allergischem Asthma kommt es aus noch nicht ganz geklärten Ursachen zur massiven Infiltration der Atemwege durch eosinophile Granulozyten und im geringeren Maße durch Lymphozyten.

Um die Zusammensetzung des Leukozyten-Infiltrats in den Atemwegen von Mäusen zu untersuchen, werden diese drei Tage nach der letzten OVA-Aerosol Exposition getötet. Die Trachea wird freigelegt und eine 24G Venen-Verweilkanüle in die Trachea inseriert. Durch diese wird das Lumen der Atemwege zweimal mit jeweils 1ml isotonischem Puffer gewaschen (Broncho-Alveoläre-Lavage). Die Gesamtzahl der Zellen, die auf diese Weise aus der Lunge herausgespült werden, wird mittels Neubauer Zählkammer mikroskopisch ausgezählt. Anschließend werden die Leukozyten der BAL mit einer Zyto-Zentrifuge der Firma Shandon auf Objektträger zentrifugiert. Die Färbung der Zellen erfolgt dann mit dem HAEME-Schnellfärbungs Kit der Firma Labor+Technik Eberhard Lehmann, wie in der Produktbeilage beschrieben. Nach erfolgter Färbung werden 300 Zellen auf dem Objektträger mikroskopisch ausgezählt und nach den gängigen Kriterien in die unterschiedlichen Leukozyten-Subpopulationen eingeteilt. Anschließend kann die prozentuale Zusammensetzung der BAL berechnet werden.

***2.4. Messung von OVA-spezifischen Antikörpern* des Isotyps *IgE und IgG1 -*** Die Sensibilisierung gegen ein Allergen kann durch die Bestimmung von Allergen-spezifischen Antikörpern der Klassen IgG1 und IgE gemessen werden. Die Quantifizierung der Allergen-spezifischen Antikörper im Serum von Mäusen erfolgt mittels indirektem ELISA, Hierzu wird den Mäusen zwei Tagen nach der letzten Applikation von OVA-Aerosol Blut aus der Schwanzvene entnommen. Nach dem Gerinnen des Bluts wird das Serum durch Zentrifugation gewonnen und im ELISA eingesetzt:

### Kurzanleitung für ELISA:

### IgE (OVA-spez.)-EL/SA:

- Platten beschichten mit 5µg/ml OVA in Beschichtungspuffer, je 100µl pro Depot (über Nacht)
- 3x waschen, jeweils 250µl pro Depot
- Nachbeschichtung mit 1% BSA in Waschpuffer, 250µl pro Depot, 1 Stunde
- 3x waschen
- Serumverdünnungen in Waschpuffer auftragen 1/3 1/10 und 1/30, 100µl pro Depot, 1 Stunde
- 3x waschen
- biotinylierten anti-IgE-Antikörper (Pharmingen, R35-72) 1/100 in Waschpuffer verdünnt auftragen, 100µl pro Depot, 1 Stunde
- 3x waschen
- Extravidin-POD (Sigma) 1/1000 in Waschpuffer verdünnt auftragen, 100µl pro Depot, 1 Stunde
- 3x waschen
- TMB-Substratlösung auftragen, 100µl pro Depot, 30min
- Reaktion abstoppen mit 50µl 1M Schwefelsäure pro Depot, Platte kann bei einer Wellenlänge von 450nm gemessen werden

### IgG1 (OVA-spez.)-ELISA:

- Platten beschichten mit 5µg/ml OVA in Beschichtungspuffer, je 100µl pro Depot (Ober Nacht)
- 3x waschen, jeweils 250µl pro Depot
- Nachbeschichtung mit 5% MMP in Waschpuffer, 250µl pro Depot, 1 Stunde
- 3x waschen
- Serumverdünnungen in Verdünnungspuffer auftragen 1/100 1/300 1/1000 und 1/3000, 100µl pro Depot, 1 Stunde
- 3x waschen
- AKP-konjugierten anti-IgG1-Antikörper (Pharmingen, X56) 1/1000 in Verdünnungspuffer verdünnt auftragen, 100µl pro Depot, 1 Stunde
- 3x waschen
- pNPP-Substratlösung auftragen, 100µl pro Depot, 30min
- Reaktion abstoppen mit 50µl 1M Natronlauge pro Depot, Platte kann bei einer Wellenlänge von 405nm gemessen werden

Auf jeder ELISA-Platte wird eine Verdünnungsreihe eines Standard-Serums mitgeführt. In diesem Standard-Serum befindet sich eine bekannte Konzentration an OVA-spezifischen Antikörpern des Isotyps IgG1 und IgE. Durch den Vergleich von Messwerten aus Mausserum mit unbekannter OVA-spez. IgG1- und IgE-Konzentration mit der Messreihe des bekannten Mausserums, kann die Konzentration in der unbekannten Probe berechnet werden.

***2.5. Behandlung von Mäusen mit Lactococcus lactis und Acinetobacte Iwoffii während der Sensibilisierung:-*** Zur Behandlung von Mäusen mit den beiden Bakterienstämmen Lactococcus lactis G121 bzw. Acinetobacter Iwoffii F78 wird diesen jeweils in narkotisiertem Zustand intranasal 10° cfu lyophilisierte Bakterien, bzw PBS als Kontrolle, jeden zweiten Tag, beginnend 10 Tage vor der ersten Sensibilisierung, über den gesamten Zeitraum der Sensibilisierungs- und Auslösephase (siehe oben: "*Sensibilisierung und Induktion von allergischem Asthma bei Mäusen mit Ovalbumin (OVA)*") verabreicht.

***2.6. Bildung und Stimulierung von menschlichen, von Monozyten abgeleiteten dendritischen Zellen (moDCs)**.* Heparinisiertes Blut von gesunden Spendern wurde mit Hilfe der Ficoll (PAA Laboratories GmbH, Pasching, Österreich) Trennungsmethode hersgestellt und die peripheren mononucleären Zellen (PBMCs) gewonnen. Anschließend wurden die Monocyten durch counter flow Elutriationszentrifugation isoliert. Die moDCs wurden dann wie bei Sallusto & Lanzavecchia, J. Exp. Med. 1994; 179:1109-1119 beschrieben hergestellt. Kurz zusammengefaßt läßt sich dieses Verfahren wie folgt darstellen. Monocyten (95% Reinheit) wurden mit 10⁶ Zellen /ml in RPMI 1640 (Cambrex, East Rutherford, USA), ergänzt mit 10% hitzeinaktiviertem fötalem Kälberserum (Biochrom AG, Bertin, Deutschland), 100 U/ml Penicillin (PAA Laboratories GmbH), 100µg/ml Streptomycin (PAA Laboratories GmbH), 500 U/ml GM-CSF (Strathmann Biotec GmbH, Hamburg, Deutschland) und 500 U/ml IL-4 ((Strathmann Biotec GmbH), wobei dir Hälfte des Mediums einschließlich der Cytokine alle 2 bis 3 Tage ausgetauscht wurde, kultiviert. An Tag 7 wurden die Zellen geerntet und in vollständigem Medium mit einer Dichte von 10⁶ Zellen/ml in 24-well Platten weiter kultiviert. Die Zellen wurden entweder unbehandelt gelassen oder mit 10 ng/ml LPS aus Salmonella enterica sv. Friedenau bzw. 10⁶ cfu/ml L.lactis G121 oder A.lwoffi, F78 behandelt.

***2.7. Expression co-stimulierender Moleküle In menschlichen moDCs*** - Nach 24 Std. Inkubation mit den unter 2.6 angegebenen Stimuli wurden die Zellen geerntet und mit Fluorochrorn-konjugierten monoklonalen Antikörpern (mAbs) für CD40, CD80, CD86 oder MHC Klasse II in Kontakt gebracht und mit 1,5% paraFormaldehyd fixiert. Die mAbs für CD40-PE und CD80-PE, MHC Klasse II-PE, IgG1-und lgG2a-PE wurden von BeckmannCoulter (Fullerton, USA) gekauft, der CD86-PE mAB wurde von BD PahrMingen (San Diego, USA) erworben. Die Zellen wurden durch die Flußzytometrie auf einem FACSCalibur und Anwendung des CeliQuest Programmes (Becton Dickinson, San Jose, USA) analysiert.

***2.8. Real-time quantitative PCR -*** Nach 3, 6 und 12 Std. Inkubation mit den oben genannten Stimuli wurde unter Verwendung des Absolutly RNA Kit von Stratagene aus den moDCs Gesamt-RNA isoliert und revers transcribiert zu cDNA unter Verwendung von Oligo(dT)₁₂₋₁₈ und Superscript reverser Transcriptase (Invitrogen). Die Transcripte wurden durch quantitative real-time PCR auf einem LightCycler 2.0 mit dem FastStart DNA Master Plus SYBR Green Kit (Roche Applied Science, Mannheim, Deutschland) gemäß den Anweisungen der Hersteller quantifiziert. Für jede Probe wurde das spezifische mRNA Vorkommen auf die Menge der mRNA von HPRT normalisiert und in frei gewählten Einheiten widergegeben. Die real-time PCRs wurden mit den Primem durchgeführt, die in der folgenden Tabelle 2 widergegeben sind:

**Tabelle 2**

| Primer | Sequenz (5'→3') |
|---|---|
| HPRT-for | GTCAGGCAGTATAATC |
| HPRT-rev | GGGCATATCCTACAAC |
| TNFα-for | CCTGTAGCCCATGTTGT |
| TNFα-rev | TTGAAGAGGACCTGGG |
| IL-12p40-for | AAAGGAAGATGGAATT |
| IL-12p40-rev | TGCTGCTTTTGACACTG |
| Delta-4-for | GGCCTGTTTTGTGACCA |
| Delta-4-rev | CGACAGGTGCAGGTGT |
| Jagged-1-for | CTGGCGGCTGGGAAGG |
| Jagged-1-rev | GAGGGCTGCAGTCATT |

### Beispiel 3: Ergebnisse der in vivo Versuche

***3.1. Asthmasymptome -*** Der am weitesten gehende Versuch zum Nachweis der biologischen Wirkung der Bakterien als Medikament oder in einer erfindungsgemäßen pharmazeutischen Zusammensetzung ist die Untersuchung der durch Allergie ausgelösten Asthmasymptome bei den Mäusen. Abb. 1 zeigt, dass eine deutlische Verbesserung der Atmungsaktivität bei den asthmatischen Mäusen durch die Behandlung mit den Bakterien Lactococcus lactis G121 und Acinetobacter Iwoffii F78 erreicht werden kann, und eine Methacholin-Konzentration, die eine 50%ige Reduktion des mittleren Ausatmungs-Luftflusses (MCH₅₀) bei acht Mäusen pro Gruppe bewirkte, sogar über dem Niveau von unbehandelten Mäusen ohne Asthma liegt.

***3.2. Entzündung In den Bronchien -*** Bei Mäusen mit Asthma reflektieren eosinophile Zellen in den Bronchien den Zustand einer asthmatischen Entzündung. Abb. 2 belegt, dass durch die Behandlung der Mäuse mit den Bakterien L.lactis G121 und A.lwoffii F78 die Zahl der eosinophilen Zellen, die in kranken Mäusen stark erhöht sind und im Normalfall fehlen, stark absinkt und nur noch geringfügig zu finden sind.

**3.3. Allergische Sensibilisierung** - Die Asthmasymptome werden in den Mäusen durch Sensibilisierung mit einem Allergen ausgelöst. Deshalb wurde untersucht, ob die bei Sensibilisierung ansteigenden IgG1-Antikörper gegen das Allergen durch die erfindungsgemäße Gabe der Bakterien beeinflusst werden. Die Tabelle dokumentiert, dass die gegen Ovalbumin gerichteten lgG1 Antikörper bei Anwendung der Bakterien L.lactis G121 und A.Iwoffi F78 im Vergleich zu den unbehandelten Mäusen abfallen. Der Anstieg der lgG1 Antikörper bei Ovalbuminsensibilisierung reflektiert eine Reaktion der T-Lymphozyten vom Typ Helfer-2 (Th2), deren Erhöhung bei Allergien zu beobachten sind. Hier zeigt die Tabelle 3, dass bei Gabe der Bakterien ein Abfall der Antikörperkonzentration zu verzeichnen ist.

**Tabelle 3**

| **Sensibilisierung** | **keine** | **OVA** | **OVA** | **OVA** |
|---|---|---|---|---|
| **Behandlung** | **keine** | **PBS** | **L.lactis G121** | **A.lwoffiF78** |
| OVA-IGE [ng/ml] | 0 | 3,5 ± 1,5 | 3,9 ± 0,7 | 4,0 ± 1,5 |
| OVA-IgG1 [mg/ml] | 0 | 3,8 ± 1,2 | 2,2 ± 1,2 | 2,5 ± 1,8 |
| OVA-lgH2a [ng/ml] | 0 | 321 ± 265 | 418 ± 264 | 245 ± 278 |

### 3.4. Stimulierung von moDCs

Die mit den Bakterien in Kontakt gebrachten moDCs wurden durch den Zusatz der Bakterien zur CD40 und CD86 Synthese stimuliert (FACS-Daten nicht gezeigt) und zwar bei unter Stimulierung mit A.Iwoffi F78 bereits bei einer Konzentration von ca 10² bis 10³ cfu/ml, bei L.lactis G121 bei einer Konzentration von ca. 10⁴ cfu/ml.

Die Expression von TNFα und IL-12 nach Stimulierung mit LPS, L.lactis G121 und A.lwoffii F78 wurde auf mRNA Ebene untersucht. Die Ergebnisse sind in Abb. 3A gezeigt. Die erhöhte Expression führe auch zu einer Erhöhung der Freisetzung der Cytokine durch die Zellen (Abb.3B).

Um die Fähigkeit der Bakterien zur Modulierung der T_{Helfer} Zell Polarisierung zu testen, wurde die Expression von Notch Liganden auf dendritischen Zellen nach der Stimulierung mit den Bakterien untersucht. Von dendritischen Zellen werden zwei Gruppen von Notch Liganden exprimiert und deren Verhältnis ist wichtig für die Polarisierung der T_{Helfer} Zellen: die Delta-Gruppe leitet die T-Zell-Polarisierung in Richtung TH₁, während die Jagged-Gruppe die T_{H}2-Antworten induziert. Der Abb. 4 ist zu entnehmen, dass die Stimulierung der Zellen mit den Bakterien eine deutliche Erhöhung der Delta-4 Expression bei gleichzeitigen Reduzierung der Jagged Expression bewirkt.

## Patentansprüche

1. Pharmazeutische Zusammensetzung zur nasalen oder inhalativen Verabreichung, enthaltend natürlich vorkommende, nicht transgene, isolierte Bakterien, ausgewählt aus der Gruppe bestehend aus Lactococcus und Acinetobacter oder Fragmente davon oder eine Mischung davon und ggf. einen pharmazeutisch akzeptablen Träger zur Vorbeugung und / oder Behandlung von allergischen oder chronischen entzündlichen Erkrankungen, ausgewählt aus IgE-abhängigen Typl Allergien oder Typ IV Allergien und chronisch entzündlichen Hauterkrankungen oder Autoimmunerkrankungen.

2. Pharmazeutische Zusammensetzung gemäß Anspruch 1, **dadurch gekennzeichnet, dass** sie als Aerosol, wässrige Lösung, Suspension, Lyophilisat oder Pulver vorliegt.

3. Mischung natürlich vorkommender, nicht transgener Lactococcus und Acinetobacter Bakterien oder Fragmente davon als Medikament zur Vorbeugung und /oder Behandlung von allergischen oder chronischen entzündlichen Erkrankungen, ausgewählt aus IgE-abhängigen Typl Allergien oder Typ IV Allergien und chronisch entzündlichen Hauterkrankungen oder Autoimmunerkrankungen, bevorzugt durch nasale oder inhalative Verabreichung.

4. Zusammensetzung gemäß Anspruch 1 oder 2 oder Medikament gemäß Anspruch 3, enthaltend eine Kombination von Lactococcus und Acinetobacter in einem Mischungsverhältnis von 5:95 bis 95:5.

5. Zusammensetzung oder Medikament gemäß einem der Ansprüche 1 bis 4, wobei die Bakterien ausgewählt sind aus Lactococcus lactis und Acinetobacter Iwoffii.

6. Zusammensetzung oder Medikament gemäß einem der Ansprüche 1 bis 5, wobei die Bakterien in vitalem oder abgetötetem Zustand vorliegen.

7. Zusammensetzung oder Medikament gemäß einem der Ansprüche 1 bis 6 zur Vorbeugung und/oder Behandlung von Heuschnupfen, Nahrungsmittelallergien, Asthma, Neurodermitis, atopische Dermatitis, Kontaktekzem, Psoriasis, Diabetes Typ 1 oder 2, multiple Sklerose, Rheumatoide Arthritis, Schilddrüsenerkrankungen wie Hashimoto Thyreoditis und Graves disease.

8. Zusammensetzung oder Medikament gemäß einem der Ansprüche 1 bis 7, das zum Vorbeugen von allergischen oder chronisch entzündlichen Erkrankungen bei Säuglingen oder Schwangeren, die auf Grund einer positiven Familienanamnese ein deutlich höheres Risiko aufweisen, eine solche Erkrankung zu entwickeln, geeignet ist und /oder zum Vorbeugen oder Behandeln von allergischen oder chronischen entzündlichen Erkrankungen bei Säuglingen oder Schwangeren, die erste Manifestationen einer solchen Erkrankung aufweisen, geeignet ist.

9. Zusammensetzung oder Medikament gemäß einem der Ansprüche 1 bis 8, zusätzlich enthaltend wenigstens ein weiteres Bakterium ausgewählt aus Micrococcus spp., Planococcus spp., Staphylococcus spp., Streptococcus spp., Lactococcus spp., Aerococcus spp., Gemella spp., Enterococcus spp., Bacillus spp., Clostridium spp., Micromonospora spp., Lactobacillus spp., Listeria spp., Erysipelothrix spp., Acinetobacter spp., Leuconostoc spp., Corynebacterium spp., Clavibacter spp., Brevibacterium spp., Propionibacterium spp., Arthrobacter spp., Curtobacterium spp., Microbacterium spp., Aureobacterium spp., Cellulomonas spp., Agromyces spp., Eubacterium spp., Pseudomonas spp., Xantomonas spp., Frateuria spp., Zoogloea spp., Escherichia spp., Enterobacter spp., Pantoea spp., Erwinia spp., Citrobacter spp., Klebsiella spp., Proteus spp., Serratia spp., Actinomyces spp., Streptomyces spp., Nocardia spp., Mycobacterium spp., Absidia spp., Wallemia spp., Eurotium spp., Acremonium spp., Aspergillus spp., Penicillium spp., Cladosporium spp., Cephalosporium spp., Fusarium spp., Mucor spp., Trichoderma spp., oder in Kombination mit Trichothecenen, Fumonisinen, Gliotoxin, Ochratoxinen, Patulin, Aflatoxinen oder Fragmenten daraus oder Mischungen davon.

10. Verfahren zur Herstellung einer pharmazeutischen Zusammensetzung oder eines Medikaments gemäß einem der Ansprüche 1 bis 8 , umfassend die Schritte:
a. Isolieren eines natürlichen, nicht transgenen Bakteriums, ausgewählt aus der Gruppe bestehend aus Lactococcus und Acinetobacter
b. Anzüchten des isolierten Bakteriums in Kultur
c. Ggf. Abtöten und/ oder Lyophilisieren des Bakteriums
d. Ggf. Fragmentieren des Bakteriums
e. Versetzen des vitalen oder ggf. abgetöteten, lyophilisierten oder fragmentierten Bakteriums mit einem pharmazeutisch akzeptablen Träger.

11. Verfahren gemäß Anspruch 10, wobei der pharmazeutisch akzeptable Träger ausgewählt ist aus Wasser, Alkohol, einer Puffer- oder Salzlösung, einer wässrig-alkoholischen Mischung, pharmazeutisch akzeptablem Pulver, gemahlenem Silikat, Stärke, Cellulose und Cellulosederivaten

## Claims

1. Pharmaceutical composition for nasal or inhalative administration, comprising naturally occurring, non-transgenic, isolated bacteria selected from the group consisting of *Lactococcus* and *Acinetobacter* or fragments thereof or a mixture thereof and, where appropriate, a pharmaceutically acceptable carrier for the prevention and/or treatment of allergic or chronic inflammatory disorders selected from IgE-dependent type I allergies or type IV allergies, and chronic inflammatory cutaneous disorders or autoimmune diseases.

2. Pharmaceutical composition as claimed in claim 1, which is in the form of an aerosol, aqueous solution, suspension, lyophilizate or powder.

3. Mixture comprising naturally occurring, non-transgenic *Lactococcus* and *Acinetobacter* or fragments thereof as medicament for the prevention and/or treatment of allergic or chronic inflammatory disorders selected from IgE-dependent type I allergies or type IV allergies, and chronic inflammatory cutaneous disorders or autoimmune diseases, preferably for nasal or inhalative administration.

4. Composition of claim 1 or 2 or medicament according to claim 3, comprising a combination of *Lactococcus* and *Acinetobacter* in a mixing ratio of from 5:95 to 95:5.

5. Composition or medicament according to any one of claims 1 to 4, wherein the bacteria are selected from *Lactococcus lactis* and *Acinetobacter Iwoffii.*

6. Composition or medicament according to any one of claims 1 to 5, wherein the bacteria are in the vital or killed state.

7. Composition or medicament according to any one of claims 1 to 6, for the prevention and/or treatment of hay fever, food allergies, asthma, neurodermatitis, atopic dermatitis, contact eczema, psoriasis, type 1 or 2 diabetes, multiple sclerosis, rheumatoid arthritis, thyroid disorders such as Hashimoto's thyroiditis and Graves' disease.

8. Composition or medicament according to any one of claims 1 to 7, which is suitable for the prevention of allergic or chronic inflammatory disorders in babies or pregnant women who, owing to a positive family history, have a distinctly increased risk of developing such a disorder, and/or is suitable for the prevention or treatment of allergic or chronic inflammatory disorders in babies or pregnant women who show the first manifestations of such a disorder.

9. Composition or medicament according to any one of claims 1 to 8, further comprising at least one bacterium selected from the group consisting of *Micrococcus spp., Planococcus spp., Staphylococcus spp., Streptococcus spp., Lactococcus spp., Aerococcus spp., Gemella spp., Enterococcus spp., Bacillus spp., Clostridium spp., Micromonospora spp., Lactobacillus spp., Listeria spp., Erysipelothrix spp., Acinetobacter spp., Leuconostoc spp., Corynebacterium spp., Clavibacter spp., Brevibacterium spp., Propionibacterium spp., Arthrobacter spp., Curtobacterium spp., Microbacterium spp., Aureobacterium spp., Cellulomonas spp., Agromyces spp., Eubacterium spp., Pseudomonas spp., Xantomonas spp., Frateuria spp., Zoogloea spp., Escherichia spp., Enterobacter spp., Pantoea spp., Erwinia spp., Citrobacter spp., Klebsiella spp., Proteus spp., Serratia spp., Actinomyces spp., Streptomyces spp., Nocardia spp., Mycobacterium spp., Absidia spp., Wallemia spp., Eurotium spp., Acremonium spp., Aspergillus spp., Penicillium spp., Cladosporium spp., Cephalosporium spp., Fusarium spp., Mucor spp.,* and *Trichoderma spp.,* or in combination with trichothecenes, fumonisins, gliotoxin, ochratoxins, patulin, aflatoxins or fragments thereof or mixtures thereof.

10. Process for producing a pharmaceutical composition or a medicament according to any one of claims 1 to 8, comprising the steps:
a. isolating a natural, non-transgenic bacterium selected from the group consisting of *Lactococcus* and *Acinetobacter*,
b. growing of the isolated bacterium in culture,
c. where appropriate killing and/or lyophilizing the bacterium,
d. where appropriate fragmenting the bacterium, and
e. mixing the vital or, where appropriate, killed, lyophilized or fragmented bacterium with a pharmaceutically acceptable carrier.

11. Process as claimed in claim 10, where the pharmaceutically acceptable carrier is selected from the group consisting of water, alcohol, a buffer solution or salt solution, a hydroalcoholic mixture, pharmaceutically acceptable powder, ground silicate, starch, cellulose and cellulose derivatives.

## Revendications

1. Composition pharmaceutique pour l'administration par voie nasale ou par inhalation, comprenant des bactéries isolées non transgéniques et natives, choisies dans le groupe constitué par les genres *Lactococcus* et *Acinetobacter,* ou des fragments de celles-ci ou un mélange de celles-ci et le cas échéant un support pharmaceutiquement acceptable pour la prévention et/ou le traitement des maladies inflammatoires allergiques ou chroniques, choisies parmi les allergies IgE dépendantes de type I ou les allergies de type IV et les affections dermatologiques inflammatoires chroniques ou les maladies auto-immunes.

2. Composition pharmaceutique selon la revendication 1, **caractérisée en ce qu'**elle se présente sous forme d'aérosol, de solution aqueuse, de suspension, de lyophilisat ou de poudre.

3. Mélange de bactéries non transgéniques natives *Lactococcus* et *Acinetobacter* ou de fragments de celles-ci sous la forme d'un médicament pour la prévention et/ou le traitement des maladies inflammatoires allergiques ou chroniques, choisies parmi les allergies IgE dépendantes de type I ou les allergies de type IV et les affections dermatologiques inflammatoires chroniques ou les maladies auto-immunes, de préférence par administration par voie nasale ou par inhalation.

4. Composition selon la revendication 1 ou 2 ou médicament selon la revendication 3, comprenant une combinaison de *Lactococcus* et *Acinetobacter* en un rapport de mélange de 5/95 à 95/5.

5. Composition ou médicament selon l'une des revendications 1 à 4, dans laquelle ou lequel les bactéries sont choisies parmi les *Lactococcus lactis* et *Acinetobacter Iwoffii.*

6. Composition ou médicament selon l'une des revendications 1 à 5, dans laquelle les bactéries se présentent à l'état vivant ou tué.

7. Composition ou médicament selon l'une des revendications 1 à 6 pour la prévention et/ou pour le traitement du rhume des foins, des allergies alimentaires, de l'asthme, de la neurodermite, de la dermatite atopique, de l'eczéma de contact, du psoriasis, du diabète de type 1 ou 2, de la sclérose en plaques, de l'arthrite rhumatoïde, des maladies des glandes endocrines comme la thyroïdite d'Hashimoto et la maladie de Graves.

8. Composition ou médicament selon l'une des revendications 1 à 7, qui est approprié(e) à la prévention des maladies allergiques ou inflammatoires chroniques chez les nourrissons ou les femmes enceintes présentant un risque nettement plus élevé de développer une telle maladie du fait d'antécédents familiaux positifs, et/ou à la prévention ou au traitement des maladies allergiques ou inflammatoires chroniques chez les nourrissons ou les femmes enceintes présentant des premières manifestations d'une telle maladie.

9. Composition ou médicament selon l'une des revendications 1 à 8, comprenant en outre au moins une autre bactérie choisie parmi les genres Micrococcus spp., Planococcus spp., Staphylococcus spp., Streptococcus spp., Lactococcus spp., Aerococcus spp., Gemella spp., Enterococcus spp., Bacillus spp., Clostridium spp., Micromonospora spp., Lactobacillus spp., Listeria spp., Erysipelothrix spp., Acinetobacter spp., Leuconostoc spp., Corynebacterium spp., Clavibacter spp., Brevibacterium spp., Propionibacterium spp., Arthrobacter spp., Curtobacterium spp., Microbacterium spp., Aureobacterium spp., Cellulomonas spp., Agromyces spp., Eubacterium spp., Pseudomonas spp., Xantomonas spp., Frateuria spp., Zoogloea spp., Escherichia spp. Enterobacter spp., Pantoea spp., Erwinia spp., Citrobacter spp., Klebsiella spp., Proteus spp., Serratia spp., Actinomyces spp., Streptomyces spp., Nocardia spp., Mycobacterium spp., Absidia spp., Wallemia spp., Eurotium spp., Acremonium spp., Aspergillus spp., Penicillium spp., Cladosporium spp., Cephalosporium spp., Fusarium spp., Mucor spp., Trichoderma spp., ou en combinaison avec des trichothécènes, des fumonisines, la gliotoxine, les ochratoxines, la patuline, les aflatoxines ou des fragments de ceux-ci ou des mélanges de ceux-ci.

10. Procédé de préparation d'une composition pharmaceutique ou d'un médicament selon l'une des revendications 1 à 8, comprenant les étapes de :
a. isolation d'une bactérie naturelle non transgénique, choisie dans le groupe constitué par *Lactococcus* et *Acinetobacter*
b. élevage en culture de la bactérie isolée
c. le cas échéant inactivation et/ou lyophilisation de la bactérie
d. le cas échéant fragmentation de la bactérie
e. transfert de la bactérie vivante ou le cas échéant tuée, lyophilisée ou fragmentée avec un support pharmaceutiquement acceptable.

11. Procédé selon la revendication 10, dans lequel le support pharmaceutiquement acceptable est choisi parmi l'eau, l'alcool, une solution tampon ou saline, un mélange aqueux alcoolique, une poudre pharmaceutiquement acceptable, un silicate broyé, l'amidon, la cellulose ou des dérivés de cellulose.
